# EUROPEAN PATENT SPECIFICATION

(11) **EP 3 850 959 B1**
(45) Date of publication and mention of the grant of the patent: **14.02.2024**
(21) Application number: 20197439.1
(22) Date of filing: 22.09.2020
(51) Int. Cl.: A24F 40/30, A24F 40/42, A24F 40/60, A61M 15/06

(54) **ELECTRONIC CIGARETTE**
ELEKTRONISCHE ZIGARETTE
CIGARETTE ÉLECTRONIQUE

(30) Priority: 12.12.2019 CN 201911273077; 12.12.2019 CN 201922228546 U
(43) Date of publication of application: 21.07.2021
(73) Proprietor: Liu, Tuanfang, 518000 Shenzhen, Guangdong (CN)
(72) Inventor: Liu, Tuanfang, 518000 Shenzhen, Guangdong (CN)
(74) Representative: Scholl, Matthias

(56) References cited:
- WO-A1-2015/135115
- WO-A1-2016/000136
- CN-A- 109 602 091
- US-A1- 2014 060 556
- US-A1- 2018 289 069
- US-B1- 10 123 568

## Description

The disclosure relates to an atomization assembly and an electronic cigarette comprising the same.

A conventional electronic cigarette includes only one atomizer equipped with only one heating element. US 10123568 B1 discloses an aerosol-generating device including multiple heating elements that are accommodated inside an e-liquid storage medium. CN 109 602 091 A describes an atomizer containing a bottom cover connected to an outer shell of the atomizer.

The disclosure also provides an electronic cigarette, comprising an atomization assembly and a battery assembly; the atomization assembly comprises an atomizer and an e-liquid tank; the atomizer comprises two or more heating elements; the e-liquid tank comprises a plurality of compartments corresponding to the heating elements in number; and the two or more heating elements are disposed in the plurality of compartments, respectively.

The battery assembly is disposed on the bottom end of the atomization assembly. The battery assembly comprises a bottom casing. The bottom casing is rotatable with respect to the battery assembly so as to switch the working mode of the atomizer.

The battery assembly comprises the top part and an electrode pair disposed on the top part. The atomization assembly comprises the bottom part and a joint disposed on the bottom part. The electrode pair is in contact with the joint for electric conduction. The bottom casing of the battery assembly is rotatable thus driving a switching printed circuit board (PCB) disposed on the top end of a rotating shaft to rotate to power on or power off the electrode pair, so as to switch the working mode of the atomizer.

The bottom casing is rotatable with respect to the battery assembly so as to switch the working mode of the atomizer to control the two or more heating elements disposed in the plurality of compartments of the e-liquid tank to work alone or simultaneously.

The atomization assembly further comprises a mouthpiece, a seal plug, a rubber gasket, a bottom cover, and a magnet. The atomizer is disposed on the rubber gasket. The rubber gasket is disposed on the bottom cover. The joint is disposed on the bottom end of the bottom cover to fix the atomizer. The bottom cover is disposed on a bottom part of the e-liquid tank to seal the e-liquid tank. The seal plug is disposed on the top part of the e-liquid tank to seal an e-liquid injection hole of the e-liquid tank; and the mouthpiece is disposed on the top end of the e-liquid tank.

The battery assembly further comprises a rotatable fixed seat, an insulation tube, an LED tube, a bolt, a decorative board, a battery, a control panel, a rubber ring, a support, and a battery case. The electrode pair is disposed on the support to supply power to the atomization assembly. The rubber ring is disposed on the support to insulate the electrode pair. The insulation tube is disposed on the top end of the rotating shaft. The rotatable fixed seat is disposed on the insulation tube to fix the rotating shaft. The switching PCB is disposed on the rotatable fixed seat and is connected to the rotating shaft. The LED tube is disposed on the bottom end of the rotating shaft. The rotating shaft is disposed in the support. The battery is soldered on the control panel and the control panel is fixed on the support. The support is disposed in the battery case. The bottom casing is disposed on the bottom end of the battery case, in contact with the LED tube to connect to and control the rotating shaft. The bolt is in threaded connection to the bottom end of the bottom casing. The LED tube is fixed in the bottom casing. The decorative board is attached to a bottom surface of the bottom casing; and the magnet is disposed on the top end of the support.

The atomization assembly comprises an atomizer and an e-liquid tank. The atomizer comprises two or more heating elements. The e-liquid tank comprises a plurality of compartments corresponding to the heating elements in number; and the two or more heating elements are disposed in the plurality of compartments, respectively. The plurality of compartments is filled with different e-liquid materials. The users can switch the working mode of the atomizer to vaporize different e-liquid materials.
FIG. 1 is an exploded view of an electronic cigarette according to one embodiment of the disclosure;
FIG. 2 is an exploded view of an atomization assembly of an electronic cigarette according to one embodiment of the disclosure;
FIG. 3 is an exploded view of a battery assembly of an electronic cigarette according to one embodiment of the disclosure;
FIG. 4 is a schematic diagram of an electronic cigarette according to one embodiment of the disclosure; and
FIG. 5 is a sectional view of an electronic cigarette according to one embodiment of the disclosure.

### DETAILED DESCRIPTION

To further illustrate, embodiments detailing an electronic cigarette are described below. It should be noted that the following embodiments are intended to describe and not to limit the disclosure.

In the disclosure, smoke materials refer to smoke oil, tobacco, tobacco and other materials used to produce smoke.

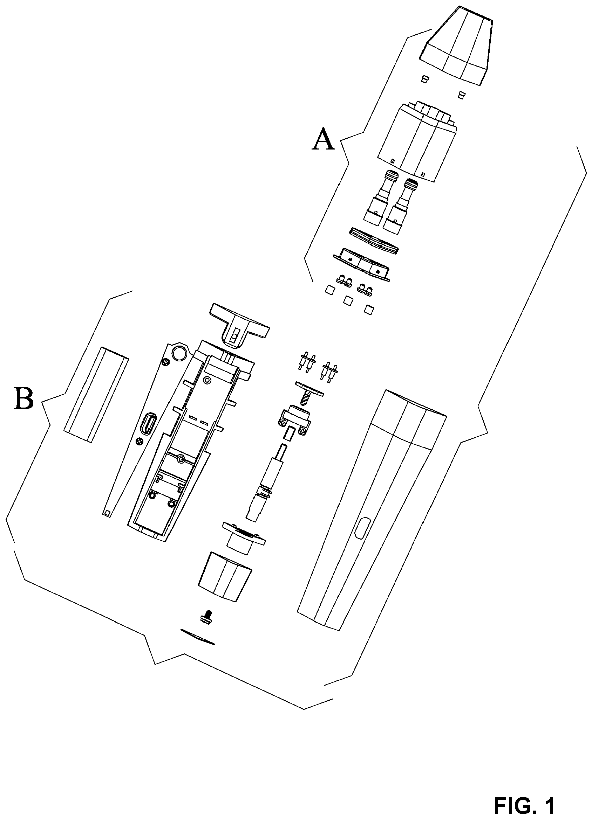

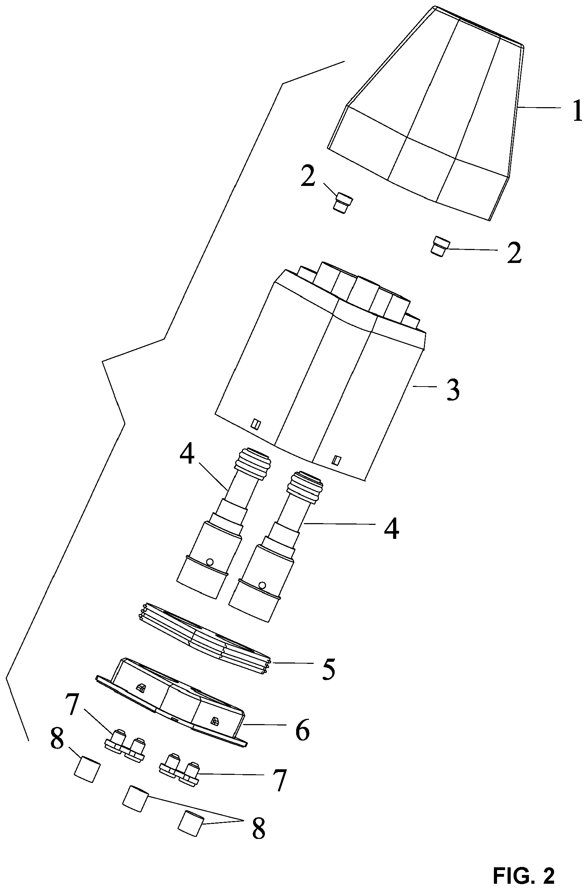

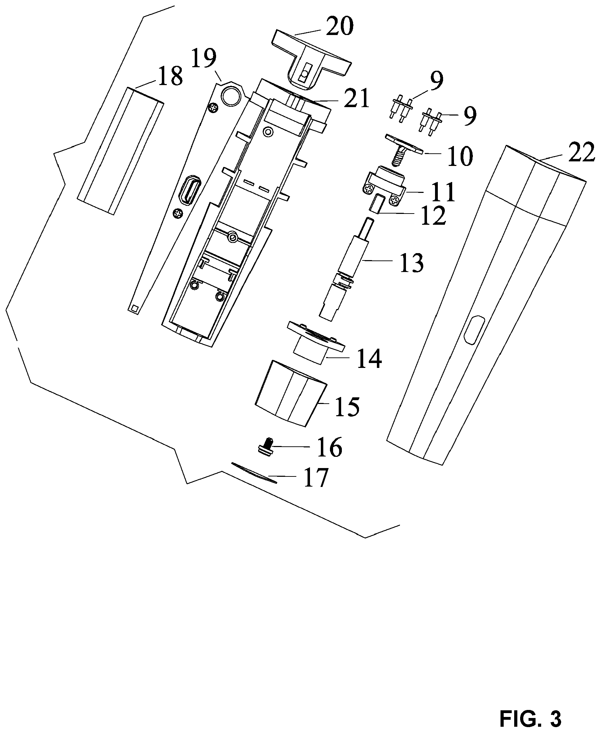

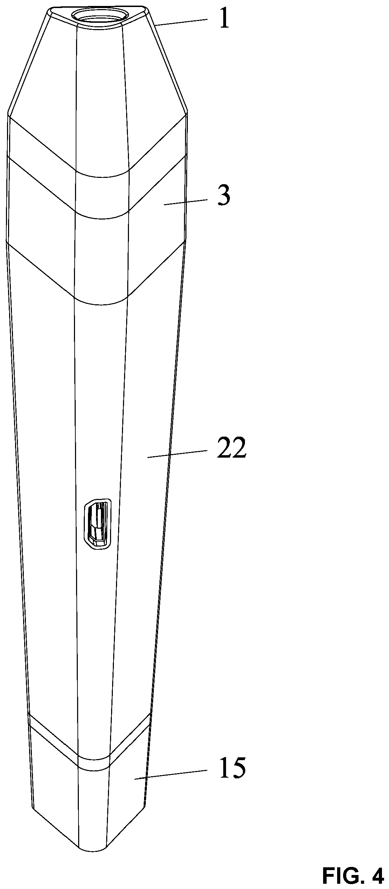

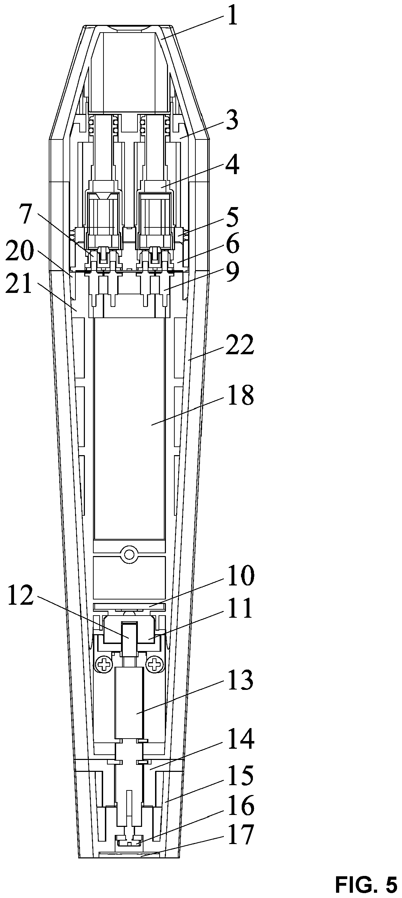

As shown in FIGS. 1-5, an electronic cigarette comprises two heating elements and an e-liquid tank 3 comprising two compartments. Specifically, the electronic cigarette comprises an atomization assembly A and a battery assembly B. The atomization assembly A a mouthpiece 1, a seal plug 2, an e-liquid tank 3, an atomizer 4, a rubber gasket 5, a bottom cover 6, a joint 7, and a magnet 8. The atomizer 4 is disposed on the rubber gasket 5. The rubber gasket 5 is disposed on the bottom cover 6. The joint 7 is disposed on the bottom end of the bottom cover 6 to fix the atomizer 4. The bottom cover 6 is disposed on a bottom part of the e-liquid tank 3 to seal the e-liquid tank. The seal plug 2 is disposed on the top part of the e-liquid tank 3 to seal an e-liquid injection hole of the e-liquid tank; and the mouthpiece 1 is disposed on the top end of the e-liquid tank 3.

The battery assembly B further comprises an electrode pair 9, a switching PCB 10, a rotatable fixed seat 11, an insulation tube 12, a rotating shaft 13, an LED tube 14, a bottom casing 15, a bolt 16, a decorative board 17, a battery 18, a control panel 19, a rubber ring 20, a support 21, and a battery case 22. The electrode pair 9 is disposed on the support 21 to supply power to the atomization assembly. The rubber ring 20 is disposed on the support 21 to insulate the electrode pair 9. The insulation tube 12 is disposed on the top end of the rotating shaft 13. The rotatable fixed seat 11 is disposed on the insulation tube 12 to fix the rotating shaft 13. The switching PCB 10 is disposed on the rotatable fixed seat 11 and is connected to the rotating shaft 13. The LED tube 14 is disposed on the bottom end of the rotating shaft 13. The rotating shaft 13 is disposed in the support 21. The battery 18 is soldered on the control panel 19 and the control panel 19 is fixed on the support 21. The support 21 is disposed in the battery case 22. The bottom casing 15 is disposed on the bottom end of the battery case 22, in contact with the LED tube 14 to connect to and control the rotating shaft 13. The bolt 16 is in threaded connection to the bottom end of the bottom casing 15. The LED tube 14 is fixed in the bottom casing 15. The decorative board 17 is attached to a bottom surface of the bottom casing 15; and the magnet 8 is disposed on the top end of the support 21. The atomization assembly A is disposed on the battery assembly B and is connected to the battery assembly B through the magnet 8.

The electronic cigarette comprises two heating elements and an e-liquid tank 3 comprising two compartments. The bottom casing 15 is rotatable with respect to the battery assembly B so as to switch the working mode of the atomizer to control the two heating elements respectively disposed in the two compartments of the e-liquid tank to work alone or simultaneously. The two compartments are filled with different e-liquid materials. The users can switch the working mode of the atomizer to vaporize different e-liquid materials.

## Claims

1. An electronic cigarette, comprising an atomization assembly (A) and a battery assembly (B);
wherein
the atomization assembly comprises an atomizer (4) and an e-liquid tank (3); the atomizer (4) comprises two or more heating elements; the e-liquid tank (3) comprises a plurality of compartments corresponding to the heating elements in number; and the two or more heating elements are disposed in the plurality of compartments, respectively;
**characterised in that**
the battery assembly (B) is disposed on a bottom end of the atomization assembly (A); the battery assembly (B) comprises a bottom casing (15), a top part, an electrode pair (9) disposed on the top part, a rotatable fixed seat (11), an insulation tube (12), an LED tube (14), a bolt (16), a decorative board (17), a battery (18), a control panel (19), a rubber ring (20), a support (21), and a battery case (22); the bottom casing (15) is rotatable with respect to the battery assembly (B); the electrode pair (9) is disposed on the support (21) to supply power to the atomization assembly; the rubber ring (20) is disposed on the support (21) to insulate the electrode pair (9); the insulation tube (12) is disposed on a top end of a rotating shaft (13); the rotatable fixed seat (11) is disposed on the insulation tube (12) to fix the rotating shaft (13); a switching PCB (10) is disposed on the rotatable fixed seat (11) and is connected to the rotating shaft (13); the LED tube (14) is disposed on a bottom end of the rotating shaft (13); the rotating shaft (13) is disposed in the support (21); the battery (18) is soldered on the control panel (19) and the control panel (19) is fixed on the support (21); the support (21) is disposed in the battery case (22); the bottom casing (15) is disposed on a bottom end of the battery case (22), in contact with the LED tube (14) to connect to and control the rotating shaft (13); the bolt (16) is in threaded connection to a bottom end of the bottom casing (15); the LED tube (14) is fixed in the bottom casing (15); the decorative board (17) is attached to a bottom surface of the bottom casing (15); and a magnet (8) is disposed on a top end of the support (21); and
the atomization assembly A comprises a bottom part and a joint (7) disposed on the bottom part; the electrode pair (9) is in contact with the joint (7) for electric conduction; the bottom casing (15) of the battery assembly (B) is rotatable thus driving the switching PCB (10) disposed on the top end of the rotating shaft (13) to rotate to power on or power off the electrode pair (9), so as to switch the working mode of the atomizer (4) to control the two or more heating elements to work alone or simultaneously.

2. The electronic cigarette of claim 1, wherein the atomization assembly (A) further comprises a mouthpiece (1), a seal plug (2), a rubber gasket (5), a bottom cover (6), and the magnet (8); the atomizer (4) is disposed on the rubber gasket (5); the rubber gasket (5) is disposed on the bottom cover (6); the joint (7) is disposed on a bottom end of the bottom cover (6) to fix the atomizer (4); the bottom cover (6) is disposed on a bottom part of the e-liquid tank (3) to seal the e-liquid tank; the seal plug (2) is disposed on a top part of the e-liquid tank (3) to seal an e-liquid injection hole of the e-liquid tank; and the mouthpiece (1) is disposed on a top end of the e-liquid tank (3).

## Patentansprüche

1. Elektronische Zigarette, umfassend eine Zerstäubungsbaugruppe (A) und eine Batteriebaugruppe (B);
wobei
die Zerstäubungsbaugruppe einen Zerstäuber (4) und einen E-Liquid-Tank (3) umfasst; der Zerstäuber (4) zwei oder mehr Heizelemente umfasst; der E-Liquid-Tank (3) eine Vielzahl von Fächern entsprechend der Anzahl der Heizelemente umfasst; und die zwei oder mehr Heizelemente jeweils in der Vielzahl von Fächern angeordnet sind;
**dadurch gekennzeichnet, dass**
die Batteriebaugruppe (B) an einem unteren Ende der Zerstäubungsbaugruppe (A) angeordnet ist; die Batteriebaugruppe (B) eine untere Umhüllung (15), einen oberen Teil, ein auf dem oberen Teil angeordnetes Elektrodenpaar (9), einen drehbaren fixierten Sitz (11), eine Isolierröhre (12), eine LED-Röhre (14), einen Bolzen (16), eine Zierplatte (17), eine Batterie (18), ein Bedienfeld (19), einen Gummiring (20), eine Halterung (21) und ein Batteriegehäuse (22) umfasst; die untere Umhüllung (15) in Bezug auf die Batteriebaugruppe (B) drehbar ist; das Elektrodenpaar (9) auf der Halterung (21) angeordnet ist, um die Zerstäubungsbaugruppe mit Strom zu versorgen; der Gummiring (20) auf der Halterung (21) angeordnet ist, um das Elektrodenpaar (9) zu isolieren; die Isolierröhre (12) an einem oberen Ende einer Drehwelle (13) angeordnet ist; der drehbare fixierte Sitz (11) auf der Isolierröhre (12) angeordnet ist, um die Drehwelle (13) zu fixieren; eine Schalt-PCB (10) auf dem drehbaren fixierten Sitz (11) angeordnet ist und mit der Drehwelle (13) verbunden ist; die LED-Röhre (14) an einem unteren Ende der Drehwelle (13) angeordnet ist; die Drehwelle (13) in der Halterung (21) angeordnet ist; die Batterie (18) in dem Bedienfeld (19) angelötet ist und das Bedienfeld (19) an der Halterung (21) fixiert ist; die Halterung (21) in dem Batteriegehäuse (22) angeordnet ist; die untere Umhüllung (15) an einem unteren Ende des Batteriegehäuses (22) in Kontakt mit der LED-Röhre (14) angeordnet ist, um eine Verbindung mit der Drehwelle (13) herzustellen und diese zu steuern; der Bolzen (16) in einer Gewindeverbindung mit einem unteren Ende der unteren Umhüllung (15) steht; die LED-Röhre (14) in der unteren Umhüllung (15) fixiert ist; die Zierplatte (17) an einer unteren Fläche der unteren Umhüllung (15) angebracht ist; und ein Magnet (8) an einem oberen Ende der Halterung (21) angeordnet ist;
und die Zerstäubungsbaugruppe A einen unteren Teil und ein an dem unteren Teil angeordnetes Gelenk (7) umfasst; das Elektrodenpaar (9) zur elektrischen Leitung in Kontakt mit dem Gelenk (7) steht; die untere Umhüllung (15) der Batteriebaugruppe (B) drehbar ist und somit die Schalt-PCB (10) antreibt, die an dem oberen Ende der Drehwelle (13) angeordnet ist, sodass sich diese dreht, um das Elektrodenpaar (9) ein- oder auszuschalten, um den Arbeitsmodus des Zerstäubers (4) umzuschalten, um die zwei oder mehr Heizelemente zu steuern, sodass diese einzeln oder gleichzeitig betrieben werden.

2. Elektronische Zigarette nach Anspruch 1, wobei die Zerstäubungsbaugruppe (A) ferner ein Mundstück (1), einen Verschlussstopfen (2), eine Gummidichtung (5), eine untere Abdeckung (6) und den Magneten (8) umfasst; der Zerstäuber (4) an der Gummidichtung (5) angeordnet ist; die Gummidichtung (5) an der unteren Abdeckung (6) angeordnet ist; das Gelenk (7) an einem unteren Ende der unteren Abdeckung (6) angeordnet ist, um den Zerstäuber (4) zu fixieren; die untere Abdeckung (6) an einem unteren Teil des E-Liquid-Tanks (3) angeordnet ist, um den E-Liquid-Tank abzudichten; der Verschlussstopfen (2) an einem oberen Teil des E-Liquid-Tanks (3) angeordnet ist, um ein E-Liquid-Einspritzloch des E-Liquid-Tanks abzudichten; und das Mundstück (1) an einem oberen Ende des E-Liquid-Tanks (3) angeordnet ist.

## Revendications

1. Cigarette électronique, comprenant un ensemble d'atomisation (A) et un ensemble batterie (B) ;
ledit ensemble d'atomisation comprenant un atomiseur (4) et un réservoir de liquide à vapoter (3) ; ledit atomiseur (4) comprenant deux éléments chauffants, ou plus ; ledit réservoir de liquide à vapoter (3) comprenant une pluralité de compartiments correspondant au nombre d'éléments chauffants ; et lesdits deux éléments chauffants, ou plus, étant disposés respectivement dans la pluralité de compartiments ;
**caractérisée en ce que**
l'ensemble batterie (B) est disposé sur une extrémité inférieure de l'ensemble d'atomisation (A) ; l'ensemble batterie (B) comprend un boîtier inférieur (15), une partie supérieure, une paire d'électrodes (9) disposée sur la partie supérieure, un siège (11) fixe pouvant tourner, un tube isolant (12), un tube LED (14), un boulon (16), un panneau décoratif (17), une batterie (18), un panneau de commande (19), un anneau en caoutchouc (20), un support (21) et un boîtier de batterie (22) ; le boîtier inférieur (15) pouvant tourner par rapport à l'ensemble batterie (B) ; la paire d'électrodes (9) est disposée sur le support (21) pour alimenter l'ensemble d'atomisation ; l'anneau en caoutchouc (20) est disposé sur le support (21) pour isoler la paire d'électrodes (9) ; le tube isolant (12) est disposé sur une extrémité supérieure d'un arbre rotatif (13) ; le siège (11) fixe pouvant tourner est disposé sur le tube isolant (12) pour fixer l'arbre rotatif (13) ; une PCB de commutation (10) est disposée sur le siège (11) fixe pouvant tourner et est raccordée à l'arbre rotatif (13) ; le tube LED (14) est disposé sur une extrémité inférieure de l'arbre rotatif (13) ; l'arbre rotatif (13) est disposé dans le support (21) ; la batterie (18) est soudée sur le panneau de commande (19) et le panneau de commande (19) est fixé sur le support (21) ; le support (21) est disposé dans le boîtier de batterie (22) ; le boîtier inférieur (15) est disposé sur une extrémité inférieure du boîtier de batterie (22), en contact avec le tube LED (14) pour se raccorder à l'arbre rotatif (13) et le commander ; le boulon (16) est en raccordement filetée avec une extrémité inférieure du boîtier inférieur (15) ; le tube LED (14) est fixé dans le boîtier inférieur (15) ; le panneau décoratif (17) est attaché à une surface inférieure du boîtier inférieur (15) ; et un aimant (8) est disposé sur un extrémité supérieure du support (21) ; et
l'ensemble d'atomisation A comprend une partie inférieure et un joint (7) disposé sur la partie inférieure ; la paire d'électrodes (9) est en contact avec le joint (7) pour la conduction électrique ; le boîtier inférieur (15) de l'ensemble batterie (B) peut tourner, entraînant ainsi la PCB de commutation (10) disposée sur l'extrémité supérieure de l'arbre rotatif (13) à tourner pour allumer ou éteindre la paire d'électrodes (9), de façon à commuter le mode de fonctionnement de l'atomiseur (4) pour commander les deux éléments chauffants, ou plus, pour qu'ils fonctionnent seuls ou simultanément.

2. Cigarette électronique selon la revendication 1, ledit ensemble d'atomisation (A) comprenant en outre un embout buccal (1), un bouchon d'étanchéité (2), un joint en caoutchouc (5), un couvercle inférieur (6), et l'aimant (8) ; ledit atomiseur (4) étant disposé sur le joint en caoutchouc (5) ; ledit joint en caoutchouc (5) étant disposé sur le couvercle inférieur (6) ; ledit joint (7) étant disposé sur une extrémité inférieure du couvercle inférieur (6) pour fixer l'atomiseur (4) ; ledit couvercle inférieur (6) étant disposé sur une partie inférieure du réservoir de liquide à vapoter (3) pour sceller le réservoir de liquide à vapoter ; ledit bouchon d'étanchéité (2) étant disposé sur une partie supérieure du réservoir de liquide à vapoter (3) pour sceller un trou d'injection de liquide à vapoter du réservoir de liquide à vapoter ; et ledit embout buccal (1) étant disposé sur une extrémité supérieure du réservoir de liquide à vapoter (3).
